(19) Europäisches Patentamt / European Patent Office / Office européen des brevets

(11) **EP 4 763 992 A1**

(12) **EUROPEAN PATENT APPLICATION**

(43) Date of publication:
24.06.2026 Bulletin 2026/26

(51) International Patent Classification (IPC):
*C12Q 1/6806* (2018.01)

(21) Application number: 24222779.1

(22) Date of filing: 23.12.2024

(52) Cooperative Patent Classification (CPC):
(C-Sets available)
**C12Q 1/6806** (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Johannes Gutenberg-Universität
Mainz
55122 Mainz (DE)

(72) Inventors:
• HELM, Mark
55126 Mainz (DE)
• SCHOBER, Benedikt
55118 Mainz (DE)

• KOPIETZ, Kevin
55116 Mainz (DE)
• WALTHER, Andreas
50823 Koel (DE)
• AKINTAYO, Cecilia Oluwadunsin
65474 Bischofsheim (DE)

(74) Representative: Patentanwälte Dr. Keller,
Schwertfeger
Partnerschaft mbB
Westring 17
76829 Landau (DE)

Remarks:
The complete document including Reference
Table(s) and the Sequence Listing(s) can be
downloaded from the EPO website

(54) **METHODS AND KITS FOR ISOLATING A TARGET NUCLEIC ACID BY IN-GEL AFFINITY ELECTROPHORESIS**

(57) 1. The present invention relates to methods and kits for isolating a target nucleic acid by in-gel affinity electrophoresis. The method comprises the steps of:

a. covalently immobilizing an anchor oligonucleotide during polyacrylamide polymerization in a polyacrylamide binding gel, wherein the anchor oligonucleotide comprises

(a) a binding element that covalently binds to acrylamide of the polyacrylamide binding gel and
(b) a first coupling nucleotide sequence that is complementary to a nucleotide sequence of a bridge oligonucleotide to hybridize the anchor oligonucleotide to the bridge oligonucleotide, wherein the bridge oligonucleotide comprises
(a) a second coupling nucleotide sequence that is complementary to the first coupling sequence of the anchor oligonucleotide and
(b) a landing site with at least one complementary target nucleotide sequence,

b. hybridization of the anchor oligonucleotide to the bridge oligonucleotide,
c. performing a polyacrylamide gel electrophoresis (PAGE) to enrich target nucleic acid by in-gel hybridization,
d. depletion of undesired species by in-gel nucleic acid depletion,
e. isolating the hybridized target nucleic acid from the binding gel.

EP 4 763 992 A1

(52) Cooperative Patent Classification (CPC): (Cont.)

C-Sets
**C12Q 1/6806, C12Q 2565/125, C12Q 2565/519,
C12Q 2565/543, C12Q 2527/125, C12Q 2523/303,
C12Q 2525/197**

**Description**

The present invention relates to methods and kits for isolating a target nucleic acid by in-gel affinity electrophoresis.

Manipulation of nucleic acids bearing specific sequences with nucleotide precision is an overarching problem in Live Sciences and may be regarded as a bottleneck on the way to clinical applications such as medicinal RNA and genome editing. Preparation, enrichment and outright purification of RNA of defined sequences, even through highly specific hybridization approaches, suffers from inherent fundamental limitations. These include, but are not limited to surface adhesion, lack of scalability, imperfect hybridization, and overall entropic effects, leading to enrichment of the desired sequence rather than its absolute purification. Hybridization techniques have been developed to address some of these limitations.

Hybridization of RNA is usually done by surface-immobilized cDNA, a technology that is used, for instance, for the enrichment of polyA RNA using immobilized oligo-dT, or for the isolation of individual small RNAs like tRNA. Typically, cDNA is immobilized on magnetic beads, allowing facile separation of hybridized material from the supernatant. An alternative method utilizes so-called chaplet columns, where each column contains a resin with a different immobilized cDNA through which a mixture of RNAs is circulated, thus allowing the isolation of several RNAs from the same biological mixture. Although the development of such advanced methods is moving in the right direction, the amount of target RNA that can be purified is limited by the surface of the material, and thus upscaling beyond single-digit microgram amounts is not easily achievable. A further drawback is that unspecific surface adhesion causes contamination of the obtained material. Thus, instead of obtaining only perfectly complementary RNA, the procedure results only in an enrichment, but it does not provide RNA with a high degree of purity. The enrichment of a specific target nucleic acid can, in principle, be potentiated by successive iterations of the procedure, which is, however, extremely demanding on resources in terms of time, cost, and amount of starting material. So far, only small amounts of RNA have been isolated with a defined, known sequence by hybridization (M. Cheng et al; "Methods for isolation of messenger RNA from biological samples", Anal. Methods, 2021, 13, 289-298).

US 6,214,187 B1 describes a method for analyzing the nucleic acid sequence of at least one target nucleic acid using gel electrophoresis, using at least one nucleic acid capture ligand immobilized in the medium and a spatial gradient of the nucleic acid denaturant. An electric field is applied while it is oriented in a direction parallel to the denaturant gradient under conditions, wherein the target sample migrates from a region of high denaturant activity to a region of low denaturant activity. The target nucleic acid binds to the immobilized capture ligand at a position within the medium relative to the binding affinity between the target and capture ligand.

Against this background, it is therefore the object of the present invention to provide methods and kits for the isolation and/or purification of at least one target nucleic acid with a defined, specific nucleic acid sequence from a complex mixture of DNAs or RNAs obtained, for instance, from cells, tissues, organisms, PCR, in-vitro transcription (IVT), solid-phase synthesis or ligations.

The object is solved by a method with the features according to claim 1 and kits that provide the components for performing said method.

The present invention is based on the idea of a covalent immobilization of oligonucleotides, preferably DNA, in a gel, typically by derivatization of the oligos with an acrylic functionality, which can be incorporated into a polyacrylamide chain as a building block during polymerization. In the polyacrylamide gel, the oligos are polymerized in a defined area, the "binding gel". For example, during the electrophoresis of nucleic acids, such as RNA mixtures, through the binding gel, RNAs hybridize with sequences that are complementary to the immobilized oligo and are retained while the rest of the sample continues to pass through the gel. RNAs of defined sequences are retained, physically separated from the mixture, and can subsequently be isolated from the gel as pure species. At the same time, undesired RNAs, such as ribosomal RNA can be eliminated. Thus, the methods according to the invention not only enable the isolation of a specific RNA/DNA, but are also suitable for the purification of nucleic acids by removing unwanted RNA/DNA species.

As shown by the present invention, it is not necessary to derivatize a complementary oligonucleotide for polymerization with an acrylic residue for each new desired target sequence. Instead, two distinct oligonucleotides with different functionalities are used, an immobilized oligonucleotide and an additional oligonucleotide that binds to the immobilized oligonucleotide and bears the target sequence. The immobilized oligonucleotide is referred to here as "anchor", wherein the additional oligonucleotide is referred to here as "bridge". The anchor oligonucleotide bears a binding element that allows binding to a gel component, i.e. acrylamide. One part of the bridge, preferably the 5' section, is complementary to the anchor oligonucleotide to allow its hybridization. The bridge oligonucleotide also contains a further region that acts as a "landing site" for the desired RNA/DNA to be isolated. The landing site at the 3' section bears the nucleotide sequence that is complementary to the target sequence of the desired RNA/DNA. An optional linker sequence can separate the anchor binding site and landing site on the bridge oligonucleotide. Preferably, the linker has a length of 8 to 12 nucleotides. In alternative embodiments, the 3' section of the bridge oligonucleotide hybridizes to the 5' section of the anchor oligonucleotide, and the 3' section of the anchor is immobilized to the gel component.

Based on this principle, permutations of different elements allow the development of different setups, which, for example, will allow the isolation of two or more RNA species with a particular purity in the same electrophoresis run, or a selective removal of unwanted RNA (such as rRNA) from nucleic acid mixtures. In some embodiments of the invention, the step of separation is carried out by repeated hybridization during migration through the binding gel. More precisely, different nucleic acids can be separately purified during one gel run using two or more consecutive binding gels in series. These consecutive binding gels may be equipped with different bridges. The method is scalable to generate large quantities of target nucleic acid, and can be easily adapted to the desired nucleic acid sequence, e.g. RNA.

In a first aspect, the present invention relates to a method for isolating at least one target nucleic acid comprising the steps of:

> a. covalently immobilizing an anchor oligonucleotide during polyacrylamide polymerization in a polyacrylamide binding gel, wherein the anchor oligonucleotide comprises
>
>> (a) a binding element that covalently binds to acrylamide of the polyacrylamide binding gel and
>> (b) a first coupling nucleotide sequence that is complementary to a nucleotide sequence of a bridge oligonucleotide to hybridize the anchor oligonucleotide to the bridge oligonucleotide, wherein the bridge oligonucleotide comprises
>> (a) a second coupling nucleotide sequence that is complementary to the first coupling sequence of the anchor oligonucleotide and
>> (b) a landing site with at least one complementary target nucleotide sequence,
>
> b. hybridization of the anchor oligonucleotide to the bridge oligonucleotide,
> c. performing a polyacrylamide gel electrophoresis (PAGE) to enrich target nucleic acid by hybridization,
> d. depletion of undesired species by in-gel nucleic acid depletion,
> e. isolating the hybridized target nucleic acid from the binding gel.

A "target nucleic acid" as used in the context of the present invention includes, but is not limited to an oligonucleotide of interest (OOI). The target nucleic acid can be any RNA or DNA of interest.

A "bridge oligonucleotide" as used in the context of the present invention relates to an oligonucleotide that comprises a coupling nucleotide sequence that is complementary to the respective coupling sequence of the anchor oligonucleotide. In preferred embodiments, hybridization of the anchor oligonucleotide to the bridge oligonucleotide is performed as in-gel hybridization.

The "landing site" as used in the context of the present invention relates to a nucleotide sequence that is complementary to a target nucleotide sequence. Typically, the landing site acts as hybridization site for a sequence within the target nucleic acid (RNA or DNA).

The present invention employs in-gel hybridization, which is able to solve two major problems in the isolation of specific RNA sequences from complex mixtures. Firstly, while surfaces are limited in their binding capacity by being two-dimensional, gels are three-dimensional, and immobilization of a cDNA can thus proceed at greater capacity. Secondly, electrophoresis enables repeated encounters of a target RNA with immobilized cDNA and subsequent retention, while nonspecific sequences are also continuously removed.

The anchor oligonucleotide as used in the context of the present invention relates to an oligonucleotide that is covalently immobilized with polyacrylamide via its binding element that is preferably present in the 5' section of the anchor oligo. The binding element couples the anchor oligo to acrylamide. In the 3' section, the anchor oligonucleotide further comprises a first coupling nucleotide sequence, either RNA or DNA, that is complementary to the second coupling nucleotide sequence of the bridge oligonucleotide. The complementary nucleotide sequence of the anchor oligonucleotide can thus hybridize to the counterpart nucleotide sequence of the bridge oligonucleotide. To omit binding of any other sequence to the anchor, the sequence of the anchor is most preferably not complementary to any RNA or DNA found in the sample at hand. This can be achieved, for instance, by using so called 'nullomer' sequences. Nullomer sequences are sequences that are completely absent in the respective organism. These can either be implemented by multiple repetition of one nullomer sequence or by alteration of different nullomer sequences. By specially designing the anchors on basis of the nullomer sequences, it can be assured that any other DNA or RNA found in the organism binds to the anchor. This is an important advantage.

In preferred embodiments, immobilization of the anchor oligonucleotide occurs in a designated zone within a polyacrylamide gel. Furthermore, it is preferred that the anchor and bridge may already be hybridized before immobilization reaction. If not, the bridge may hybridize to the anchor during the polyacrylamide gel electrophoresis through in-gel hybridization. Alternatively, one can also hybridize the bridge with the target RNA and the bridge-target hybrid is then hybridized during PAGE through in-gel hybridization. Another alteration may that bridge, anchor and target sequence are already hybridized before immobilization reaction. In short, hybridization may occur before immobilization of anchor or during PAGE, but not between immobilization and PAGE.

After hybridization of the anchor oligonucleotide to the bridge oligonucleotide, a PAGE is performed in order to enrich target nucleic acid by in-gel hybridization. In addition, undesired species, such as rRNA, are depleted by in-gel nucleic acid depletion. The hybridized target nucleic acid is enriched and can then be isolated from the binding gel. In preferred embodiments, hybridization of the bridge oligonucleotide may also be performed directly with the target nucleic acid followed by a PAGE. The bridge-target complex is then bound by the anchor oligonucleotide. This offers the advantage of breaking possible secondary structures of the target nucleic acid.

The binding gel used in the method and kits of the present invention is preferably a semi-denaturing gel to immobilize anchor oligonucleotides by co-polymerization through acrylamide, i.e. in the absence of denaturing agents such as urea. Such semi-denaturing gels contain urea in a lower concentration that still enables stable hybridization of any of the sequences (e.g. 0.2 M). The binding gel acts as a separate zone within the polyacrylamide gel with covalently bound anchor oligonucleotides.

In preferred embodiments, the binding element of the anchor oligonucleotide that covalently binds to an acrylamide residue is selected from the group of a thiol group or any other nucleophilic group conjugated to bis-acrylamide, Acrydite™ modification, acrylamide or any other group that can be polymerized into an acrylamide gel. It is apparent that any suitable method to couple the anchor oligo to a gel component of the binding gel will work in the context of the present invention.

In a preferred embodiment, the first coupling nucleotide sequence of the anchor oligonucleotide and the second coupling nucleotide sequence of the bridge oligonucleotide comprises between 30 and 40 nucleotides for the anchor oligonucleotide and between 50 and 200 nucleotides for the bridge oligonucleotide.

In order to eliminate undesired nucleic acids and to increase the purity of the target nucleic acid, it is preferred that the PAGE comprises at least one run with inversed polarity.

In a preferred embodiment, the 5' section of the bridge oligonucleotide comprises the second coupling nucleotide sequence that is complementary to the first coupling sequence of the anchor oligonucleotide and the 3' section of the bridge oligonucleotide comprises the landing site of the at least one complementary target nucleic acid sequence of the at least one target nucleic acid.

In an alternative embodiment, the 3' section of the bridge oligonucleotide comprises the second coupling nucleotide sequence that is complementary to the first coupling sequence of the anchor oligonucleotide and the 5' section of the bridge oligonucleotide comprises the landing site of the at least one complementary target nucleic acid sequence of the at least one target nucleic acid.

In a further embodiment, the landing site of the bridge oligonucleotide contains multiple complementary target nucleic acid sequences for the same or different target nucleic acids. This enables the simultaneous isolation and/or purification of several different target nucleic acids.

In a further preferred embodiment, the bridge oligonucleotide is directly or indirectly bound to at least one further complementary bridge oligonucleotide, wherein the complementary target sequence of the target nucleic acid is present on one of the further complementary bridge oligonucleotides. In preferred embodiments, the bridge oligonucleotide is hybridized to a third oligonucleotide which is hybridized to a fourth and optionally a fifth oligonucleotide. The last oligonucleotide (for example the fourth or fifth oligonucleotide) can bear the complementary target nucleic acid sequence.

In some embodiments, the anchor oligonucleotides can be mixed with at least two different bridge oligonucleotides, preferably two or more bridge oligonucleotides that each bear individual different landing sites in order to isolate different target nucleic acids. For example, the bridge oligonucleotide can hybridize through the complementary nucleic acid sequence of the anchor oligonucleotide. At the other end of the bridge oligonucleotide, a third oligonucleotide can be hybridized with a complementary nucleic acid sequence, wherein a fourth oligonucleotide hybridizes with complementary nucleic acid sequence of the third oligonucleotide and the third or fifth oligonucleotide. By means of this method, multiple oligonucleotides can be bound at once with bi-complementary oligonucleotides. Reverse electrophoresis eliminates undesired RNA, while target RNA is enriched by inter-hybridization.

In further preferred embodiments, the bridge oligonucleotide can bear multiple landing sites for different target nucleic acids. Each landing site has a unique sequence that is complementary to the specific target nucleic acid.

As shown herein, the present invention allows the isolation of RNA species with a defined nucleic acid sequence obtained from any cells, tissues or organisms or through any technique like PCR, IVT, solid-phase synthesis or ligations. The methods and kits of the invention can also be used to purify specific RNAs in large amounts, for example by removing undesired RNA. As an example, the methods of the invention can be used for the production of therapeutic siRNAs or mRNAs agents. Furthermore, the methods of the invention can be used for the isolation of specific mRNAs from biological samples, either for the purpose of analysis such as quantification of mRNA or for the determination of its modifications, or the preparation field with the aim of using them for therapy or vaccination. A high degree of purity of the RNA can be obtained and the system is also scalable, i.e., the samples obtained can be implemented quantities between nanograms and milligrams. Large scale applications are admissible since the methods also allow large quantities to be processed.

The present invention also relates to kits for isolating at least one target nucleic acid. Such a kit comprises:

a. an anchor oligonucleotide which comprises

(a) a binding element that covalently binds to acrylamide and
(b) a first coupling nucleotide sequence that is complementary to a nucleotide sequence of a bridge oligonucleotide,

b. at least one bridge oligonucleotide which comprises

(a) a second coupling nucleotide sequence that is complementary to the first coupling sequence of the anchor oligonucleotide and
(b) a landing site with at least one target nucleotide sequence.

In a preferred embodiment, the kit comprises a nucleic acid preservation buffer. Preferably, the preservation buffer consists of a buffering agent that comprises phosphate, tris, borate, citrate and glycine. Preferably, the buffering agent has a pH ranging from 4-9 and a concentration ranging from 0.1M - 2M, a monovalent and divalent salt comprising sodium chlorate, lithium chlorate, potassium chlorate, magnesium chlorate or any combination thereof. Preferably, the salt has a concentration ranging from 0.01M - 0.5M. The chelating agent includes, but is not limited to ethylenediaminetetraacetate acid (EDTA), ethylene glycol-bis-tetraacetic acid, cyclohexane diaminotetraacetate (CDTA), diethylenetriaminepentaaceticacid (DTPA), tetraazacycylododecanetetraacetic acid (DOTA), tetraazacyclotetradecanetetraacetic acid (TETA), deszferioximine. Preferably, the chelating agent has a concentration ranging from 0.01 M to 0.5 M. A preferred preservation buffer comprises 5.3M $(NH_4)_2SO_4$, 19mM EDTA, 24mM Na-Citrate tribasic dihydrate.

In a further embodiment, the binding element of the anchor oligonucleotide in the kit is selected from the group consisting of thiol group or any other nucleophilic group conjugated to bis-acrylamide, Acrydite™ modification, acrylamide or any other group that can be polymerized into an arylamide gel.

In a preferred embodiment, the kit comprises at least two bridge oligonucleotides bearing landing sites for different target nucleic acids. Multiple oligonucleotides can be hybridized to the bridge oligonucleotide in order to purify nucleic acids with different target nucleic acid sequences.

In a preferred embodiment, the anchor oligonucleotide is a synthetic DNA comprising the nucleic acid sequence of SEQ ID NO: 1, 2, 3, 4, 5,8, 11 coupled to a chemical residue.

In a preferred embodiment, the bridge oligonucleotide is a synthetic DNA comprising the nucleic acid sequence of SEQ ID NO: 6, 7, 9, 10, 12, 13, 14.

The present invention is further illustrated in the following examples. By no means shall the present invention be limited to these specific examples. It is apparent for a person skilled in the art that any feature, methods or process step can be combined with others in order to arrive at embodiments that are not specifically described herein, but encompassed by the scope of the present invention.

EXAMPLES

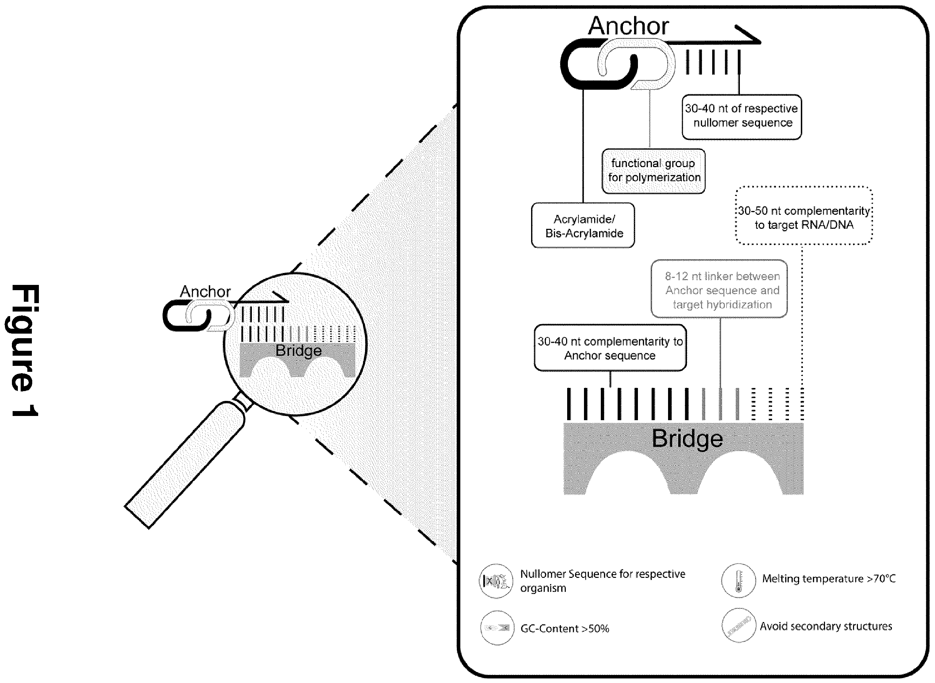

An anchor oligonucleotide comprising a binding element that covalently binds to acrylamide of a polyacrylamide binding gel has been used. The 3' section of the anchor oligonucleotide comprises nucleic acid sequences that are complementary to nucleic acid sequences of the 5' section of the bridge oligonucleotide. The 3' section of the bridge oligonucleotide comprises the landing site of at least target nucleic acid sequence. This principle is outlined in Figure 1.

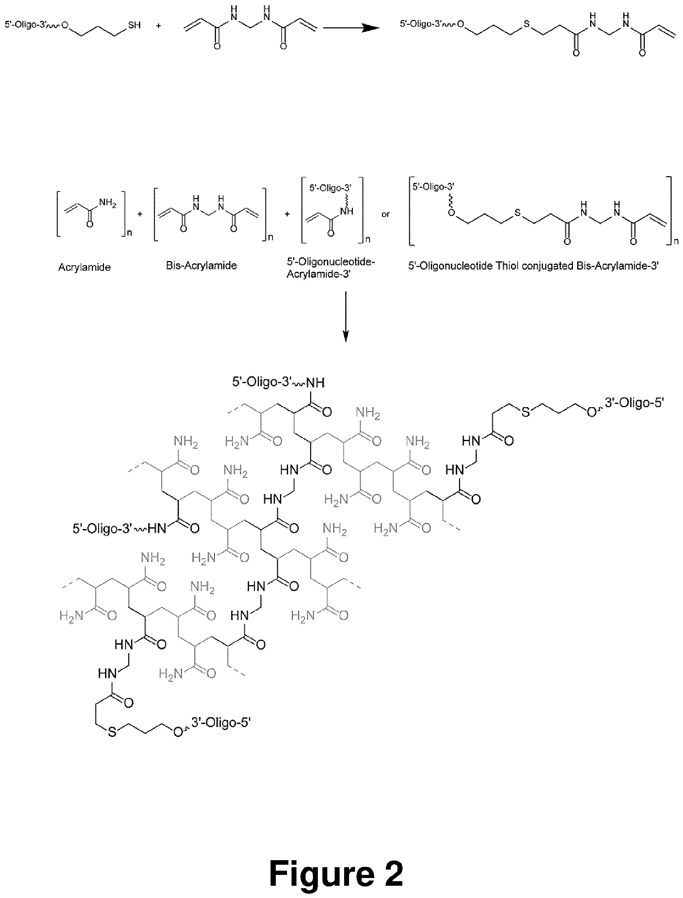

Coupling of the anchor oligo acrylamide/bis-acrylamide can be done, for instance, via a thiol residue to generate a 5'-oligonucleotide thiol conjugated bis-acrylamide as outlined in Figure 2.

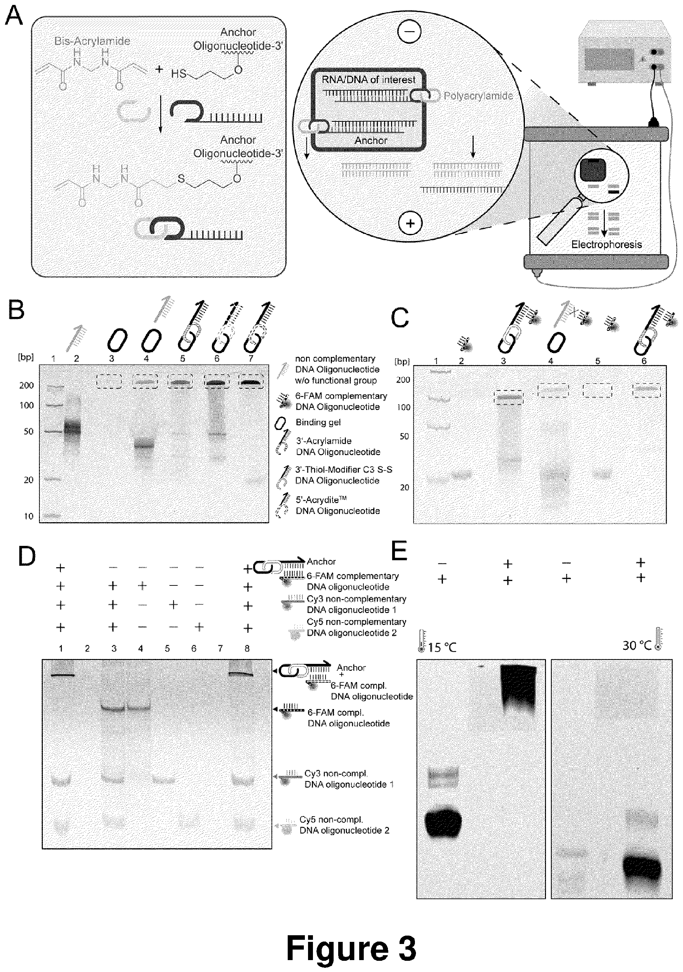

It was reasoned that retaining a complementary RNA in a gel would require only modest amounts of immobilized cDNA. Therefore, a separate zone was used as a "binding-gel" (Figure 3A), rather than covering an entire lane within a conventional polyacrylamide gel electrophoreses (PAGE) setup. The first attention was directed at the required chemistry. Four options have been tested, of which a thiol-containing DNA, derivatized in a Michael addition with bisacrylamide, is shown in Figure 3A. Figure 3B shows a comparison with commercial "acrydite"-oligonucleotide, and an in-house synthesized acrylamide-conjugate, all of which were successfully immobilized in the binding gel of a bipartite PAGE, as shown by gel-red staining after electrophoresis. In contrast, in an intended negative control, co-polymerization of a plain DNA oligonucleotide led to little, albeit detectable retention, while the vast majority of the DNA migrated normally, i.e. out of the binding gel. Subsequent electrophoresis of a fluorescent-labeled, complementary RNA (cRNA) across the binding gel resulted in quantitative retention of the latter (Figure 3B) in conventional TBE buffer. Control omission experiments in Figure 3C show the requirement for copolymerization of an acryl-conjugated oligo for retention of a fluorescent labeled complementary RNA, whereas non-complementary RNA was not retained. Simultaneous electrophoresis of three different oligonucleotides resulted in the selective retention of only the complementary species (Figure 3D).

A section of the parameter space delineated by temperature, ionic strength, and length of the hybridization sequence, was probed to balance robustness of the retention versus the possibility of eluting the RNA sample from the binding gel. As an applicable outcome, it has been found that a 20mer cRNA can be robustly retained by PAGE at 15°C (Figure 3E), and the same can be eluted from the binding gel by shifting to 30°C. Longer complementary sequences could also be hybridized, but could not be cleanly removed at higher temperatures.

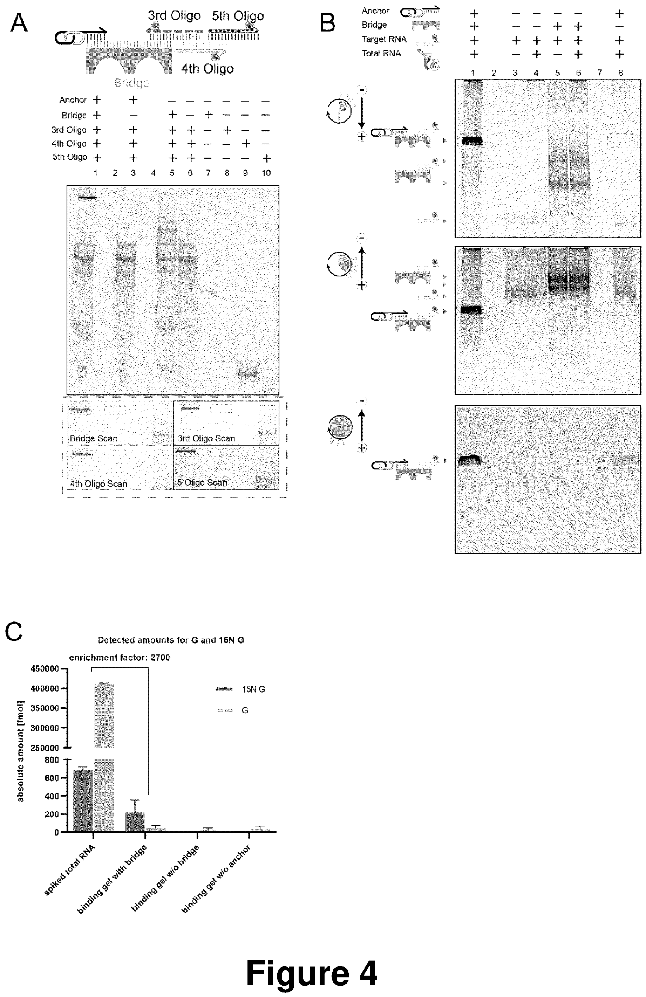

The results of in-gel hybridization are shown in Figure 4. As a terminology, the covalently immobilized oligo was termed the "anchor", onto which a "bridge" was hybridized (each marked by a corresponding symbol in Figure 4). The 5' section of the bridge is complementary to the anchor, while the remainder at the 3' extremity extends beyond the anchor sequence and now offers a landing site for hybridization of further oligos of interest (OOI). It could then be demonstrated that further bridges can be used in multiple landing sites to construct nucleic acid complexes with multiple oligos. Figure 4 shows a corresponding experiment where three differentially fluorescent labeled oligos were in-gel hybridized. As a central control, omission of the bridge prevents retention of the third oligo. Figure 4A shows fluorescent scans of the gel at different wavelengths to visualize the differentially labeled oligos. Apart from considerations about capacities for loading nucleic acid material onto the gel, so far, no experimental evidence has been encountered that would argue against extending such constructs with an indefinite number of bridges.

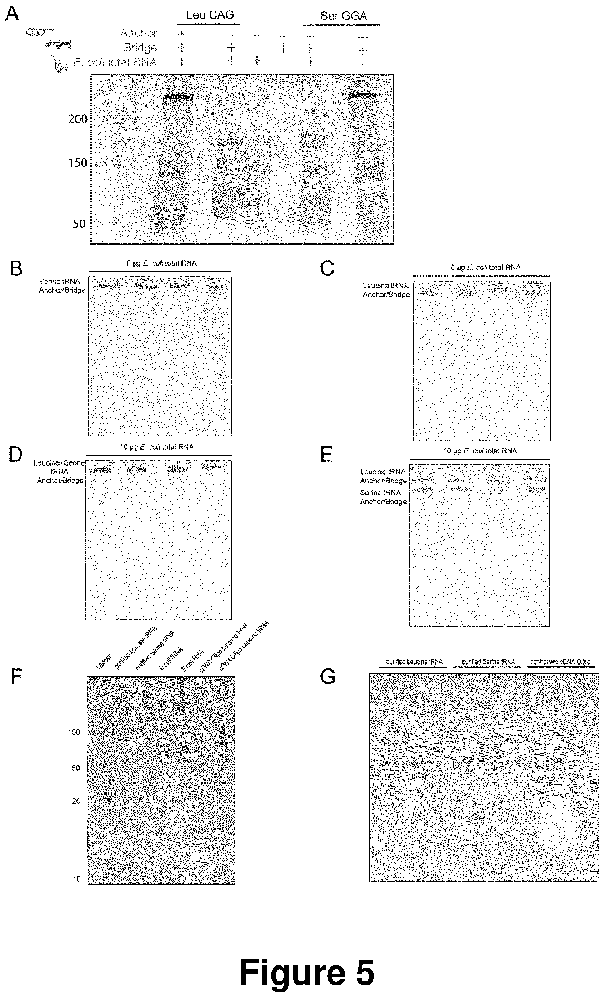

As an example, a bridge was designed with a landing site that permits the isolation of an OOI from a biological mixture. Specific tRNA iso-acceptors were isolated from an aliquot of total tRNA. To address two specific tRNAs from total *E. Coli* tRNA one separately, the same anchor, but a different bridge, designed to fit the respective OOI was used. Figure 5A shows hybridization experiments of anchor, bridges, and total tRNA after native PAGE and gel red staining. Subsequently, the electrophoresis was continued with reversed polarity, causing the unbound tRNA to leave the gel through the pockets used for the initial loading (Figure 5B). The binding gels were then excised, and nucleic acids eluted as specified in the methods section. After DNA digestion the isolated tRNA was subjected to RNAseq, in order to compare purify relative to material isolated by using cDNA immobilized on magnetic beads. While the previous efforts yielded tRNA preparations whose corresponding RNAseq data mapped to their cognate sequence with 95-98% in general, mapping yields of up to -99% could be reached.

The setup allows for the isolation of two specific tRNAs from the same binding-gel (Figure 5D). As a variation, lanes containing two binding gels were cast as different zones, each with its own specific bridge. In this setting (Figure 5E), the tRNAs were successfully isolated in different binding gels of the same lane in the gel. This setup could be extended to comprise larger numbers of RNAs (specifically: tRNAs) to be isolated in a single experimental run. The isolation was then upscaled to milligram scale, by isolating 50-70 $\mu$g of a single tRNA sequence from 2.8 mg total tRNA in. The material consumption of the upscaled experiment compared favorable against the magnetic beads approach, as the consumption comprised 1.3 nmol of anchor, 1 nmol of bridge, some sterile plastic and PAGE reagents.

The technology was then applied to more complex tasks, namely to the isolation of viral RNA sequences from a total eukaryotic RNA preparation. The OOI was an sfRNA from dengue virus, a ~300 nt long viral RNA. To be able to track progress in optimization and purification during method development, a stable-isotope-labeled version of the OOI was synthesized by *in vitro* transcription using solely 15N-labeled guanosine triphosphate in addition to conventional UTP, CTP and ATP. In later control experiments, RNA isolates to the nucleoside level, can be quantified by LC-MS for their 15N guanosine level to assess purity. A similar IVT, obtained with conventional GTP, was Cy5-labeled at its 3'-end.

Dye-labeled IVT was then mixed with total RNA from HEK cells and reisolated using a bridge sequence previously verified for efficient binding. Figure 4B shows fluorescent scans at three stages of the purification procedure. Frame 1 shows an overlay of fluorescence from the FAM-labeled bridge and the Cy5-labeled OOI in an omission assay after 2.5 hours of electrophoresis. The red band corresponds to the free OOI in lanes 3,4 and 8, while the green-yellow bridge gives rise to two bands in lanes 5 and 6, corresponding to the free bridge and a hybrid with the OOI. Binding gels were cast in lanes 8 (labeled in blue) and lane 1, where the fluorescence of bridge and OOI all accumulate, showing the retention of the OOI in the binding gel through the bridge. Electrophoresis was then continued with inversed polarity, and frame 2 shows the result of reversed migration after another 2.5 hours. While all bands have moved relative to frame 1 in lanes 2-7, fluorescence in lane 1 was still restricted to the binding gel. That held true in frame 3, taken after additional inversed electrophoresis overnight, showing that all nucleic acids had eluted from the gel through the loading pockets, except for anchor, bridge and OOI in the binding gel. From the binding gel in lane 1, nucleic acids were extracted, DNA was digested, and the was RNA submitted to RNAseq analysis.

The same experiment was also conducted with SILIS-labeled IVT, and the recovered RNA was analyzed by LC-MS for its content and ratio of $^{15}$N guanosine and $^{14}$N guanosine, the latter originating from native RNA "contaminant". A comparison to starting material allowed to calculate an enrichment factor, as well as to detect background noise resulting from nonspecific adhesion of RNA to polyacrylamide during electrophoresis. As shown in Figure 4C, the ratio dramatically reverted from the starting mixture to the isolated OOI, namely by an enrichment factor of 2700. An analysis of material from a binding gel without anchor showed 14N-G levels similar to the isolated OOI, suggesting nonspecific adhesion as potential source in both cases.

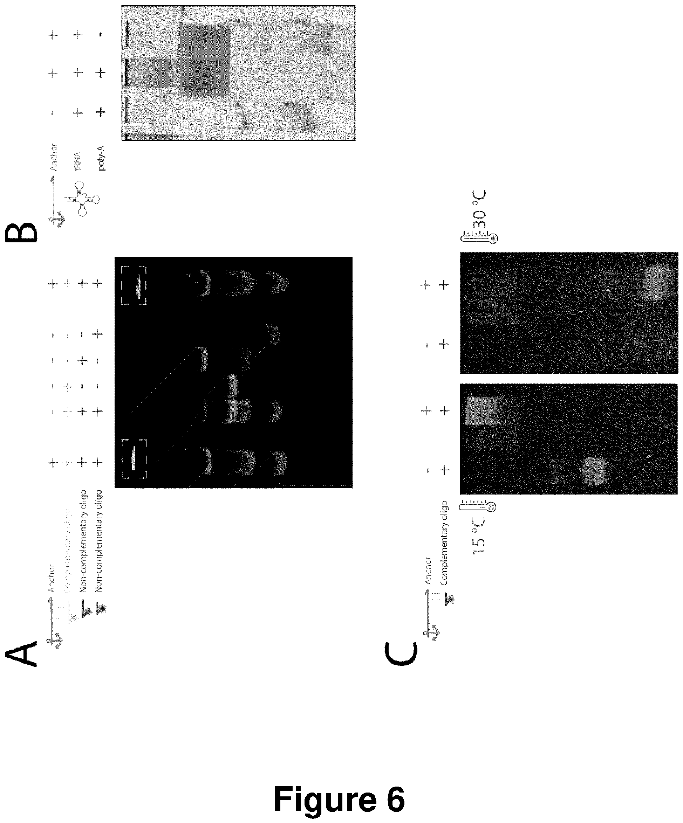

As shown in Figure 6, 180 pmol of acrylamide DNA or thiol-C3 modifier oligonucleotide was polymerized into a 10% native polyacrylamide gel to form the so-called anchor represented by the green box. The thiol-C3 modifier oligo was previously derivatized for 1 h at 37 °C with tris(2-chlorehyl)phosphate by a Michael addition to N,N'-methylenebisacrylamide, making it polymerizable.

To demonstrate sequence-specific binding, a mixture of three different oligonucleotides, each containing 3 pmol, was separated by electrophoresis (see Figure 6A). Only the complementary oligonucleotide labeled with 6-FAM (yellow) was bound by the anchor, while the migration of the non-complementary oligonucleotides (magenta or red) was not affected.

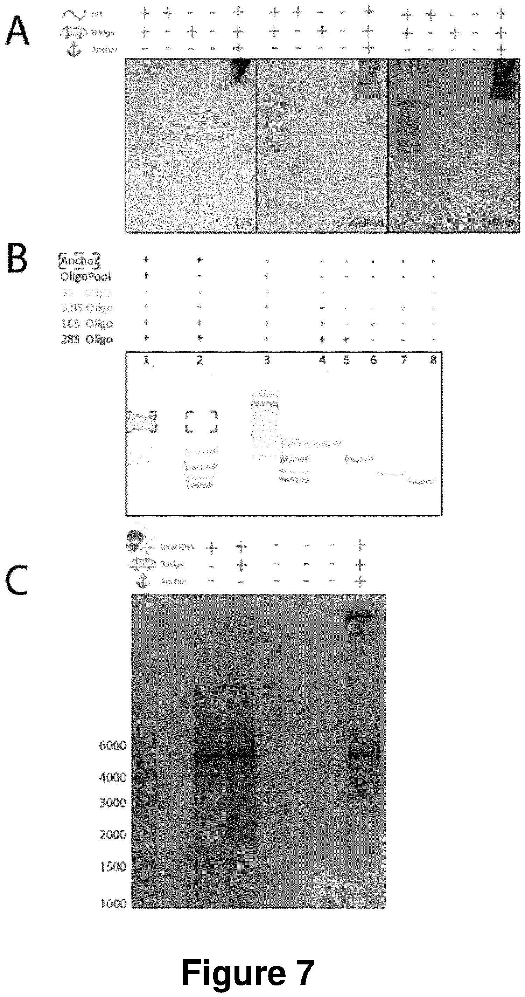

This system has now been transferred to a biological example (Figure 7). For this purpose, the complementary poly-A sequence was added to a transfer RNA (tRNA) using a poly-A polymerase. Subsequently, 5 $\mu$M of the complementary anchors were polymerized into a 10% native polyacrylamide gel and checked for binding by electrophoresis (Figure 7B). The bands were then visualized using GelRed staining. Binding could also be shown using the tRNA, as the complementary tRNA was bound by the anchor, while the non-complementary tRNA showed the same migration behavior as a tRNA that does not encounter the anchor. Furthermore, it could be shown that this binding is reversible by increasing the temperature (Figure 7C). 200 pmol of a complementary Cy5-labeled oligo (red) showed complete binding to the anchor (green) under cooled conditions. When the temperature of the electrophoresis buffer was increased to 30 °C, the binding could be quantitatively resolved.

In Figure 7A, 10 $\mu$M of the anchor was polymerized into a 7.5% native polyacrylamide gel. 7 pmol of an mRNA prepared by in-vitro transcription (IVT) was hybridized with 10 pmol of the corresponding bridge in the presence of SSC buffer (1x) with the temperature program below (Table 1) before native loading buffer (12% (v/v) glycerol, 2% (v/v) Tris-borate EDTA in water) was added. Controls were prepared in the same way with the respective absence of IVT or the bridge.

The merge image of the Cy5 and GelRed scans shows that the Cy5-labeled bridge is completely bound by the anchor. It can also be shown that the hybridization of the bridge with the IVT is quantitative and thus slows down the migration speed compared to the non-hybridized band.

Table 1: Hybridization program of the bridge and IVT mRNA

| 30 sec | 90 °C |
|--------|-------|
| 30 sec | 85 °C |
| 30 sec | 80 °C |
| 30 sec | 75 °C |
| 30 sec | 70 °C |
| 30 sec | 65 °C |
| 30 sec | 60 °C |
| 30 sec | 55 °C |
| 30 sec | 50 °C |
| 30 sec | 40 °C |
| 30 sec | 30 °C |
| 30 sec | 20 °C |

One potential application of the system is the selective removal of unwanted RNA from a mixture.

Figure 7B shows an exemplary removal of rRNA represented by short, labelled RNA oligonucleotides wherein each oligonucleotide represents one rRNA species. Lane 1 shows the binding of all oligonucleotides in the binding gel which contains an excess of 173 oligonucleotides comprehensively spanning the entirety of the rRNA sequences. In contrast, omitting the Oligo Pool in lane 2 prevents binding of all oligonucleotides. Prehybridization of Oligo Pool with the oligonucleotides in the absence of anchor results in a shift of all oligonucleotides in lane 3 relative to lane 4. As a biological example, the removal of 18S ribosomal RNA (rRNA) from eukaryotic extracted RNA was shown here. 2 pmol of the respective bridges were hybridized with total RNA from HEK cells, as mentioned in the previous paragraph. This mixture and additional controls were then loaded onto a 3%/0.5% (polyacrylamide/agarose) composition gel into which 10 $\mu$M Anchor was previously polymerized.

Figure 7C shows that the 18S rRNA could be selectively removed from the mixture, as this lane shows no band at ~1800 nucleotides. In comparison, it can also be seen that the hybridization of the bridge to the 18S rRNA results in a deceleration of the respective band.

DESCRIPTION OF THE FIGURES

FIG. 1 shows the principle of the hybridization technology according to the present invention. An anchor oligonucleotide comprises a binding element that covalently binds to acrylamide of a polyacrylamide binding gel. The 3' section of the anchor oligonucleotide comprises nucleic acid sequences that are complementary to nucleic acid sequences of the 5' section of the bridge oligonucleotide. The 3' section of the bridge oligonucleotide comprises the landing site for at least one target nucleic acid.

In order to bind to acrylamide/bis-acrylamide, the anchor oligonucleotide bears a functional group for polymerization. About 30 - 40 nucleotides bear the complementary nucleic acid sequences to bind to the bridge oligonucleotide. On the bridge oligonucleotide, 30 - 40 nucleotides are complementary to the respective nucleotide sequence of the anchor oligonucleotide. 30 - 50 are complementary to the target RNA or DNA. In a preferred embodiment, a linker that comprises between 8 - 12 nucleotides between the anchor sequence and target hybridization sequence can be incorporated.

The gc-content is preferably > 50% to obtain a melting temperature > 70° C. Secondary structures are avoided.

FIG. 2 illustrates the chemistry of coupling the anchor oligonucleotide via a thiol residue to acrylamide/bis-acrylamide.

The first reaction describes the addition of a thiol-conjugated oligonucleotide to bis-acrylamide to form an oligonucleotide with an acrylamide functionality. More generally, this reaction describes the addition of a nucleophile to an $\alpha,\beta$-unsaturated carbonyl, also known as Michael addition reaction. The second reaction shows an example how the acrylamide-conjugated oligonucleotides may be incorporated into the polyacrylamide gel matrix formed by acrylamide and bis-acrylamide during the radical chain-growth polymerization. Of note, the depicted product is only one possible outcome and only a section since the nature of the polymerization reaction is random.

FIG. 3 illustrates that oligonucleotides can be sequence-specifically bound via acrylamide polymer-conjugated complementary oligonucleotides. **(A)** Left panel: Schematic representation of in-gel immobilization chemistry of anchor oligonucleotides by covalent conjugation during polyacrylamide polymerization. The acryl functionality is here conjugated by Michael addition of a thiol-modified oligonucleotide to bis-Acrylamide. Right panel: Schematic illustration of non-covalent immobilization of and RNA or DNA of interest by in-gel hybridization during PAGE. **(B)** Covalent polymerization of DNA oligonucleotides with different conjugation chemistry. Binding gels were either prepared without any DNA oligonucleotide, with a DNA oligonucleotide without any functional group or with DNA oligonucleotides of the same sequence containing acrylamide functions of different origins (as indicated). As control, the DNA oligonucleotide without any function was loaded as sample. Gel was stained using GelRed™. **(C)** In-gel immobilization via hybridization requires complementarity of the oligonucleotide (yellow) and the copolymerized anchor. Lanes from left to right: Ladder; target oligo (yellow); copolymerized anchor (blue) and complementary target; target and non-copolymerized, noncomplementary anchor; empty binding gel (no anchor) and target; alternative chemistry for copolymerized anchor (black) and complementary target. **(D)** Complementary oligonucleotide (yellow) was selectively bound from a mixture of non-complementary oligonucleotides (red, magenta). **(E)** High amounts of 20mer complementary oligonucleotide can be bound at low temperature (15°C) and subsequently eluted by switching to warm TBE buffer (30°C).

FIG. 4 shows that an introduction of bi-complementary oligonucleotide leads to efficient retention and purification of target sequence. **(A)** Multiple oligonucleotides can be bound at once with bi-complementary oligonucleotides in lane 1 while omitting of the bridge (yellow) in lane 2, which connects the 3rd oligonucleotide to the anchor, results in no binding of the latter oligonucleotides. Lower panel shows single scans of the respective oligonucleotides. **(B)** Reverse electrophoresis eliminates undesired RNA. First panel shows electrophoresis of Cy5-labelled IVT with binding occurring in the presence of its corresponding FAM-labelled bridge. Controls are loaded in the middle as indicated above. Second panel shows the same gel after inverting the polarities for the same duration as in forward motion. Third panel shows the gel after overnight electrophoresis with inverted polarities (FAM: yellow, Cy5: red, GelRed: green). **(C)** Target RNA was enriched by in-gel hybridization. LC-MS/MS results of purified 15N stable isotope-labelled IVT in the presence of total RNA using a nullomer sequence as anchor. First panel shows absolute amounts of detected guanosine and 15N guanosine for binding gels with or without the bridge or without any immobilized sequence as well as the state in input material. Enrichment factor is indicated for samples purified in the presence of the bridge.

FIG. 5 shows hybridization experiments of anchor, bridges, and total tRNA after native PAGE and gel red staining.

FIG. 6 shows that complementary sequences can be bound and eluted again. **(A)** shows the binding of a complementary DNA oligo (yellow) from a mixture of 2 other non-complementary DNA oligos (magenta and red). **(B)** Poly-A tRNA can be bound in the binding gel. **(C)** Large amounts of 20mer complementary oligonucleotide can be bound at low temperature (15°C) and then eluted by switching to warm TBE buffer (30°C).

FIG. 7 shows possible biological applications of the methods of the invention. **(A)** By introducing the bridge, a wide variety of sequences can be bound without having to resynthesize the anchor oligo, as shown here using an IVT. **(B)** Exemplary removal of rRNA represented by short, labelled RNA oligonucleotides wherein each oligonucleotide represents one rRNA species. **(C)** shows the application of selective removal of certain sequences from a sequence mixture.

For example, rRNA can be removed from a total RNA sample.

MATERIAL AND METHODS

**Plasmid digestion:**

For in-vitro transcriptions respective plasmid (see below) containing the desired sequence downstream of a T7-promoter was linearized using the designated restriction enzyme (FastDigest Xbal, ThermoFischer Scientific, USA) according to manufacturer's protocol. Subsequent purification was performed using phenol/chloroform extraction. To the reaction volume, 0.5 volume of each phenol (Carl Roth, Germany) and chloroform (Sigma Aldrich, Germany) was added and after centrifugation the upper aqueous phase was separated followed by another extraction with phenol/chloroform mixture. Afterwards, both organic phases were combined and additionally extracted with 0.25 volume of purified water. The aqueous phases were combined and twice extracted with equal volumes of chloroform, before precipitated with two volumes of ethanol (Carl Roth, Germany) and ammonium acetate (Sigma Aldrich, Germany) to an end concentration of 0.5 M.

**In-vitro transcription:**

In-vitro transcription was performed using HiScribe® T7 High Yield RNA Synthesis Kit (NEB) following supplier's protocol with 1 $\mu$g of linearized plasmid incubated at 37 °C overnight. Stable isotope labelling of RNA was performed by exchanging GTP with 15N-labelled GTP (Silantes). Afterwards, DNA was digested at 37 °C for one hour by adding 10 units of DNase I-XT (NEB) in respective reaction buffer to a total volume of 100 $\mu$L. Reaction mixture was purified using Monarch® RNA Cleanup Kit (500 $\mu$g) (NEB) according to manufacturer's protocol with an elution volume of 100 $\mu$L purified water.

**Preparation of eukaryotic RNA**

Human hepatocarcinoma Huh7 cells were incubated at 37 °C, 5% CO2 on either 10-cm or 15-cm dish depending on different experimental requirements. Cellular confluence was maintained below 90% to make sure that all cells were grown in a monolayer. Cells were cultured in Dulbecco's Modified Eagle Medium (DMEM, Sigma Aldrich, Germany) medium supplemented with 10 % fetal calf serum (FCS, Sigma Aldrich, Germany), non-essential amino acids, 100 U/ml penicillin and 100 $\mu$g/ml streptomycin. After a wash with 1x PBS, Trypsin/EDTA was employed to detach cells before splitting and seeding. 3 X 106 Huh7 cells were seeded in 15-cm dish the day before infection. These cells were infected by DENV-2 New Guinea C (NGC) with multiplicity of infection (MOI) of 10 for 2h in 37 °C, 5% CO2 incubator. Medium was changed after 2h and total RNA was harvested 48h post infection with 750 $\mu$l of TRIzol™ Reagent (Invitrogen, USA).

**Preparation of E. coli tRNA**

E. coli total RNA was obtained from the E. coli Keio parent strain (BW25113) cultured in standard M9 medium (6.8 g/l $Na_2HPO_4$, 3 g/l $KH_2PO_4$, 0.5 g NaCl, 1 g/l $NH_4Cl$, 2 mM $MgSO_4$, 0.1 mM $CaCl_2$, 0.4% glucose) at 37°C and 190 rpm. Cells were grown until $OD_{600}$=1.8 was reached and RNA was subsequently isolated using TRI Reagent© (Sigma-Aldrich, Germany) according to the manufacturer protocol. 10 $\mu$g of E. coli total RNA hybridized with 10 pmol of the corresponding bridge was loaded on a gel containing a binding gel with 200 pmol of the corresponding anchor.

**Hybridization of RNA/DNA**

RNA or DNA was hybridized in of hybridization buffer prepared from 5x stock (150 mM HEPES pH 7.5 (Carl Roth, Germany), 500 mM potassium acetate (Carl Roth, Germany). The mixture was hybridized using a temperature gradient starting with a denaturing step at 90°C for 2 min followed by by a stepwise temperature reduction (5 °C every 1 min) until the gradient reached room temperature.

**Magnetic beads purification of E. coli tRNA**

Specific tRNAs were hybridized with the respective cDNA oligonucleotide (IBA GmbH, Germany) carrying a 3' 6-FAM label and a 5' biotin moiety. 10 $\mu$g of total RNA were mixed with 10 pmol of oligonucleotide in the presence of 5$\times$ saline-sodium citrate (SSC) buffer. Hybridization was ensured using a temperature step gradient program which starts with a denaturation step for 3 min at 90 °C followed by a stepwise temperature reduction (5 °C every 1 min) until the gradient reached room temperature. Samples were then incubated for 30 min at 25°C and 300 rpm with 25 $\mu$L Hydrophilic

Streptavidin Magnetic Beads (New England Biolabs, USA) which were prepared beforehand according to the manufacturer protocol. Supernatant was then removed afterwards by placing the tubes on a magnetic rack. The pellet was washed once with 5x SSC buffer and 3x with 0.1× SSC buffer. Next, the pellet was resuspended in RNase/DNase free water. The bound RNA was eluted at 75 °C for 3 min. After placing on a magnetic rack, the RNA containing supernatant was saved and subjected to a DNA digestion to remove the cDNA oligonucleotide. Therefore, 2 U of DNase I-XT (New England Biolabs, USA) were incubated with the corresponding buffer for 30 min at 37 °C. At last, this mixture was PAGE-purified, bands were cut out after staining with GelRed (Biotium, USA) and visualization via Typhoon Trio imager (excitation: 532 nm, emission filter: 610 BP30, (General Electric (GE Healthcare), USA)). RNA was obtained using the crush and soak method followed by ethanol precipitation.

**Polyacrylamide gel preparation:**

For the polyacrylamide stock solutions with varying concentrations Rotiphorese Gel 40 (19:1) (Carl Roth) was diluted with purified water and 10x Tris-Borat-(EDTA)-solution (Rotiphorese Carl Roth) to a final volume of 1 L. Gels were cast and run using Biometra Eco-Maxi system EBC for 1 mm gels (AnalytikJena).

Polyacrylamide gels containing at least one binding gel were cast in a three-step procedure. In the first step, an adequate volume of polyacrylamide stock solution with the desired concentration was mixed with a 10% (m/V) aqueous solution of ammonium peroxydisulfate (Carl Roth) to a final concentration of 0.1% (V/V) followed by 0.06% (V/V) of TEMED (Carl Roth). Immediately after pouring the solution between the glass plates, a plastic spacer from the CBS LSG-400-20 NA adjustable vertical electrophoresis device (C.B.S. Scientific, San Diego, USA) (15 mm * 1 mm), that was previously cut to have the desired length, was inserted until its tip was submerged roughly 1 cm in the gel solution. Spacers were fixed using clips. After complete polymerization, the spacer was removed, remaining gel residues from the now formed pocket were removed by using round filter paper Typ MN 615 (Carl Roth) followed by rinsing with water. Excess of liquid was removed with round filter paper. In the second step, 30 μL of the same polyacrylamide stock solution was mixed with 20 μL of thiol-en reaction. Polymerization was initiated by addition of 0.5 μL 10% (m/V) aqueous solution of ammonium peroxydisulfate (Carl Roth) and 3 μL of a 1:100 dilution of TEMED (Carl Roth). This mixture was transferred into the previously formed pocket followed by a layer of isopropyl alcohol (Carl Roth). After polymerization, excess of isopropyl alcohol was removed using round filter paper. Finally, the last layer was cast as described in the first step completing the gel by insertion of the comb.

**Different polymerization chemistries:**

To show binding gels with different polymerization chemistries, five binding gels were casted as follows. 80 pmol of an acryl-functional oligonucleotide, commercial Acrydite™ oligonucleotide (IDT, USA) or sequence without any functional group were polymerized in a total volume of 50 μL of 10% PAGE solution with ammonium peroxydisulfate to a final concentration of 0.1% (V/V) followed by the addition of 0.06% (V/V) TEMED. The oligonucleotide with a 3' thiol modifier C3 S-S (IDT, USA) was conjugated beforehand to bis-acrylamide (Sigma-Aldrich, Germany) in the presence of 4 μM bis-acrylamide (Sigma-Aldrich, Germany), 50 mM TCEP (MedChemExpress, USA) and 10 mM Tris-HCl buffer pH 8.5 (Tris, Carl Roth, Germany; pH adjusted using HCl) in a total reaction volume of 20 □L. The reaction mixture was incubated at 37 °C for 1 h and used without further purification. Additionally, as negative control a binding gel without any sequence was cast. 10 μL of FastRuler Ultra low range DNA ladder (ThermoFisher Scientific, USA) as well as oligonucleotide containing no functional group was loaded as samples. Gel was run at constant 65 mA for 1 h and stained with 3x GelRed (Biotium, USA) for 15 min followed by visualization as previously described.

**Cy5-labelling of IVT:**

IVT was labelled in a two-step procedure. First, 25 μg of IVT product was adenylated using 600 U of Yeast Poly(A) Polymerase (Jena Bioscience) in the presence of 1 mM 2'-azido-2'-dATP (Jena Bioscience) with its respective reaction buffer in a total volume of 50 μL. Reaction was performed for 1:30 h at 37 °C followed by purification with RNA Clean & Concentrator-5 (Zymo Research) according to manufacturer's protocol. Secondly, Cy5 was clicked to purified RNA using strain-promoted click-chemistry in the presence of 6.7 mM DBCO-Cy5.5 (Jena Bioscience) in a total volume of 50 μL 10x PBS pH 7.4 (ThermoFisher Scientific) at 37 °C for 1:30 h. Purification was performed using Monarch RNA Cleanup Kit (50 μg) (NEB).

**Reversed electrophoresis:**

For visualizing the principle of reversed electrophoresis, 3.2 pmol of Cy5 labelled sfRNA IVT spiked into 2.7 μg HEK total RNA and hybridized to 6.4 pmol of its respective complementary oligonucleotide (sfRNA bridge, see table below) as described above. Mixture was applied to a 5% PAGE with two binding gels containing 6.2 μM anchor (Acrylamide-Oligonucleotide, see table below) in a volume of 50 μL. As additional controls, samples were equally prepared without total RNA, only with complementary oligonucleotide and IVT or without oligonucleotide. Last sample was additionally applied to a binding gel lane. Gel was run at constant 65 mA for 2:30 h, followed by visualization between the glass plates using a Typhoon Trio+ (Cy5 ex: 633 nm em: 670 nm BP, FAM ex: 488 nm em: 520 nm BP). Afterwards, polarities were switched and run for 2:30 h, followed by another visualization step. Lastly, gel was run overnight with reversed polarities and stained for 15 min with 3x GelRed (Biotium) and visualized a last time. (ex: 532 nm, em: 619 nm BP)

**Purification of spike-in IVT for LC-MS/MS:**

For each sample, 600 ng (4.1 pmol) of 15N stable isotope-labelled sfRNA IVT was spiked to 2.7 μg of HEK total RNA. Hybridization to its respective complementary oligonucleotide (7.2 pmol, sfRNA bridge Nullomer 1 human, see table below) was performed as described above. As negative control, one sample was treated in the absence of the complementary oligonucleotide. Samples were applied to a 7.5% native PAGE with four binding gels containing the thiol-en reaction and one binding gel without thiol-en reaction. Gel was cast as described above. To visualize electrophoresis, 2 μL of 6x Tritrack loading dye (ThermoFisher Scientific) was also applied. Electrophoresis was carried out at constant 65 mA until xylencyanol has migrated to the lower end of the gel followed by electrophoresis with reversed polarities overnight. Gel was stained with 3x GelRed (Biotium) for 15 min under slight shaking. Binding gels were cut using surgical scalpels (No 22, Swann-Morton), smashed and soaked in 400 μL of 1x DNAse I-XT reaction buffer (NEB). After a freezing step at -80 °C, 8 U of DNAse I-XT (NEB) were added and samples were shaken overnight at 15 °C followed by addition of another 2 U of DNAse I-XT and incubation at 37 °C for 30 min. Solutions were filtered using Nanosep centrifugal devices 0.45 μm (Cytiva), precipitated and resuspended. Concentrations were determined using a Nanodrop One (ThermoScientific).

**LC-MS/MS analysis:**

For LC-MS/MS analysis 300 ng of each sample was digested in a total volume of 20 μL using an enzyme mixture containing 10 U benzonase (Sigma-Aldrich), 0.2 U snake venom phosphordiesterase from *C. adamanteus* (Worthington), 0.2 U bovine intestine phosphatase (Sigma-Aldrich), 200 ng Pentostatin (Sigma-Aldrich) and 0.6 U nuclease P1 from *P. citrinum* (Sigma-Aldrich) in a buffer containing 5 mM Tris (pH 8) and 1 mM magnesium chloride. Reaction was performed for two hours at 37 °C and quenched by diluting to 50 μL. To determine the background, above reaction was performed in the absence of RNA. A 1:50 dilution of non-purified IVT/total RNA mixture was equally digested for comparison. To all samples, 50 ng of 13C stable isotope-labelled nucleosides from *S. cerevisiae* was added for internal calibration. Analysis was executed on an Agilent 1260 series LC with an Agilent 6470B Triple Quadrupole mass spectrometer containing an electrospray ion source (gas temperature 300 °C, gas flow 7 L/min, nebulizer pressure 60 psi, sheath gas temperature 400 °C, sheath gas flow 12 L/min, capillary voltage 3000 V). Separation was performed using a Synergi Fusion RP18 column (250 × 2.0 mm, 4 □M particle size, 80 Å pore size; Phenomenex) with two solvents. Solvent A consisted of 5 mM ammonium acetate (Merck) adjusted to pH 5.3 using acetic acid (Honeywell Chemicals) while buffer B was LC-MS grade acetonitrile (VWR Chemicals) with addition of 1% (V/V) purified water. The gradient started with 100% solvent A followed by linear increase of solvent B to 8% within 10 min, which was raised to 40% solvent B after 20 min. Initial conditions were restored within 3 min and held for additional 10 min. The flow rate was constant at 0.35 mL/min. UV traces at 254 nm were registered using a diode array detector. Nucleosides were detected using dynamic multiple reaction monitoring (dMRM) in positive mode (mass transitions: G 284.0 -> 152.0; 15N G 289.0 -> 157.0; Am 282.0 -> 136.0; Cm 258.0 -> 112.1; Gm 298.0 -> 152.0; $m^1G$ 298.0 -> 166.0; $m^{22}G$ 312.0 -> 180.0; $m^{26}A$ 296.0 -> 164.1; $m^2A$ 282.0 -> 150.1; $m^2G$ 298.0 -> 166.1; m5C 258.0 -> 126.1; $m^6A$ 282.0 -> 150.1; $m^7G$ 2980 -> 166.1). Quantification was performed as described (Stefanie Kellner, Antonia Ochel, Kathrin Thüring, Felix Spenkuch, Jennifer Neumann, Sunny Sharma, Karl-Dieter Entian, Dirk Schneider, Mark Helm, Absolute and relative quantification of RNA modifications via biosynthetic isotopomers, Nucleic Acids Research, Volume 42, Issue 18, 13 October 2014, Page e142). Signals for modifications found in the digestion mixture alone were subtracted from the detected signals in the purified samples. Amount of guanosine in IVT/total RNA mixture was determined using the UV signal adjusted for the signal resulting from the internal calibration as well as from 15N labelled guanosine. Enrichment factor was defined with following equation:

$$E_f = \frac{ratio \left(\frac{15N\,G}{G}\right) of\ purified\ samples}{ratio \left(\frac{15N\,G}{G}\right) of\ in\ total\ RNA}$$

**Oligonucleotides**

The following table summarizes the nucleic acid constructs used in the present invention such as anchor, bridge or probe.

**Oligonucleotides**

| Name | Sequence (5'→3') | SEQ ID NO |
|---|---|---|
| Acrylamide-Oligonucleotide | TTTTTTTTTTTTTTTTTTTTTAGCGCTTATGTTTTTTTTTTTTTTTTTTTTTTAGCGCTTATGTTTTTTTTTTTTTTTTTTTTT-Acrylamide | 1 |
| Acrydite Oligonucleotide Fig 1 | Acrydite^TM-<br>TTTTTTTTTTTTTTTTTTTTTAGCGCTTATGTTTTTTTTTTTTTTTTTTTTTTAGCGCTTATGTTTTTTTTTTTTTTTTTTTTT | 2 |
| Thiol Oligo Oligonucleotide Fig 1 | TTTTTTTTTTTTTTTTTTTTTAGCGCTTATGTTTTTTTTTTTTTTTTTTTTTTAGCGCTTATGTTTTTTTTTTTTTTTTTTTTT-C3-Thiol | 3 |
| Non complementary oligonucleotide w/o functional group Fig 1B | GGCACCACCCCGGTGAACAGCTCCTCGCCCTTGCTCACCATGGTTGTGGAAGGGGGGTGTTTGGTGCCTGGGTTACATA | 4 |
| Thiol-Anchor Nullomer 1 human | CGCTCGACGTACGCTCGACGTACGCTCGACGTACGCTCGACGTA-C3-Thiol | 5 |
| sfRNA bridge | 6FAM-<br>AAAAAAAAAAAAAAAAAAAAAACATAAGCGCTAAAAAAAAAAAAAAAAAAAAAGGCTGCTCACGGGGTCTC CTCTAACCTCTAGTCC | 6 |
| sfRNA bridge Nullomer 1 human | TACGTCGAGCGTACGTCGAGCGTACGTCGAGCGTACGTCGAGCGACGAACGGTCGGGGGTCTCCTCTAACCTCTAGTCC | 7 |
| Thiol Nullomer *E.coli* Anchor | CTAGGAACTTCTCTAGGAACTTCTCTAGGAACTTCTCTAGGAACTTCT-C3-Thiol | 8 |
| tRNA Ser (GGA) *E. coli* Nullomer 1 Bridge | AGAAGTTCCTAGAGAAGTTCCTAGAGAAGTTCCTAGCCGTGACCCTAGGGTGAGGGGGGGGATTCGAACCCCCGATACGTTGC | 9 |
| tRNA Leu (CAG) *E. coli* Nullomer 1 Bridge | AGAAGTTCCTAGAGAAGTTCCTAGAGAAGTTCCTAGCCGTGACCCTAGGCGAGGGGGGGGGACTTGAACCCCCACGTCCGTAA | 10 |
| Nullomer 2 *E. coli* Anchor | TAGGACACTCAATAGGACACTCAATAGGACACTCAA-C3-Thiol | 11 |
| tRNA Ser (GGA) *E. coli* Nullomer 2 Bridge | TTGAGTGTCCTATTGAGTGTCCTATTGAGTGTCCTACCGTGACCCTAGGGTGAGGGGGGGGATTCGAACCCCCGATACGTTGC | 12 |
| tRNA Leu (CAG) *E. coli* Nullomer 2 Bridge | TTGAGTGTCCTATTGAGTGTCCTATTGAGTGTCCTACCGTGACCCTAGGCGAGGGGGGGGGACTTGAACCCCCACGTCCGTAA | 13 |
| Bridge for in-gel hybridization probes | 6FAM-<br>AAAAAAAAAAAAAAAAAAAAAACATAAGCGCTAAAAAAAAAAAAAAAAAAAAAGGCTGCTCACTTGCTCCCATGGTTGTGG | 14 |
| In-Gel Hybridization Probe 2 | Cy5-CCACAACCAUGGGAGCAACCACAACCAUGGGAGCAA | 15 |
| In-Gel Hybridization Probe 3 | Rhodamine6G-GACTAGCCTTCAGAGTTGCTCCCATGGTTG | 16 |

(continued)

| Name | Sequence (5'→3') | SEQ ID NO |
|---|---|---|
| In-Gel Hybridization Probe 4 | AttoRho101-CTCTGAAGGCTAGTCTATGCGCATACGACT | 17 |
| Cy5 non-complementary DNA oligonucleotide 2 | Cy5-ACTCGACAGATAC | 18 |
| Poly-T binding probe | AAAAAAAAAAAAAAAAAAAAAAAAAAAAAAAAAAAAAAAA | 19 |
| 5S rRNA mimicking Oligo | Cy5-AUGGGAGACCGCCUGGGAAU | 20 |
| 5.8S rRNA mimicking Oligo | 6-FAM-GGACACAUUGAUCAUCGACACUUCG | 21 |
| 18S rRNA mimicking Oliqo | Cy5-CACCCGAGAUUGAGCAAUAACAGGUCUGUGAUGC | 22 |
| 28S rRNA mimicking Oliqo | 6-FAM-AAGUGGCGUUCAGCCACCCGAGAUUGAGCAAUAACAGGUC | 23 |

**Plasmid**

As template for sfRNA *in-vitro* transcription, the plasmid pGL3 DENV2 NGC sfRNA1 Rluc was used (SEQ ID NO: 24).

**Claims**

1. A method for isolating at least one target nucleic acid comprising the steps of:

   a. covalently immobilizing an anchor oligonucleotide during polyacrylamide polymerization in a polyacrylamide binding gel, wherein the anchor oligonucleotide comprises

      (a) a binding element that covalently binds to acrylamide of the polyacrylamide binding gel and
      (b) a first coupling nucleotide sequence that is complementary to a nucleotide sequence of a bridge oligonucleotide to hybridize the anchor oligonucleotide to the bridge oligonucleotide, wherein the bridge oligonucleotide comprises
      (a) a second coupling nucleotide sequence that is complementary to the first coupling sequence of the anchor oligonucleotide and
      (b) a landing site with at least one complementary target nucleotide sequence,

   b. hybridization of the anchor oligonucleotide to the bridge oligonucleotide,
   c. performing a polyacrylamide gel electrophoresis (PAGE) to enrich target nucleic acid by in-gel hybridization,
   d. depletion of undesired species by in-gel nucleic acid depletion,
   e. isolating the hybridized target nucleic acid from the binding gel.

2. The method according to claim 1, wherein the hybridization of the anchor oligonucleotide to the bridge oligonucleotide is performed as in-gel hybridization.

3. The method according to any one of claims 1 to 2, wherein the binding element of the anchor oligonucleotide is selected from the group consisting of a thiol group or any other nucleophilic group conjugated to bis-acrylamide, Acrydite™ modification, acrylamide or any other group that can be polymerized into an acrylamide gel.

4. The method according to any one of claims 1 to 3, wherein the first coupling nucleotide sequence of the anchor oligonucleotide and the second coupling nucleotide sequence of the bridge oligonucleotide comprises between 30 and 40 nucleotides for the anchor oligonucleotide and between 50 and 200 nucleotides for the bridge oligonucleotide.

5. The method according to any one of claims 1 to 4, wherein the PAGE comprises at least one run with inversed polarity to eliminate undesired nucleic acid.

6. The method according to any one of claims 1 to 5, wherein the 5' section of the bridge oligonucleotide comprises the second coupling nucleotide sequence that is complementary to the first coupling sequence of the anchor oligonucleotide and the 3' section of the bridge oligonucleotide comprises the landing site of the at least one complementary target nucleic acid sequence of the at least one target nucleic acid.

7. The method according to any one of claims 1 to 6, wherein the landing site of the bridge oligonucleotide contains multiple complementary target nucleic acid sequences for the same or different target nucleic acids.

8. The method according to any one of claims 1 to 7, wherein the bridge oligonucleotide is directly or indirectly bound to at least one further complementary bridge oligonucleotide, wherein the complementary target sequence of the target nucleic acid is present on one of the further complementary bridge oligonucleotides.

9. The method according to any one of claims 1 to 8, wherein the anchor oligonucleotide is mixed with at least two different bridge oligonucleotides bearing different landing sites for isolation of different target nucleic acids.

10. The method according to any one of claims 1 to 9, wherein the target nucleic acid is selected from the group consisting of DNA, RNA or derivatives thereof, and the method includes the elimination, purification or enrichment of the target nucleic acid. Elimination of rRNA

**11.** A kit for isolating at least one target nucleic acid, comprising:

    a. an anchor oligonucleotide which comprises

        (a) a binding element that covalently binds to acrylamide and
        (b) a first coupling nucleotide sequence that is complementary to a nucleotide sequence of a bridge oligonucleotide,

    b. at least one bridge oligonucleotide which comprises

        (a) a second coupling nucleotide sequence that is complementary to the first coupling sequence of the anchor oligonucleotide and
        (b) a landing site with at least one target nucleotide sequence.

**12.** The kit according to claim 11, further comprising a nucleic acid preservation buffer.

**13.** The kit according to any one of claims 11 to 12, wherein the binding element of the anchor oligonucleotide is selected from the group consisting of thiol group or any other nucleophilic group conjugated to bis-acrylamide, Acrydite™ modification, acrylamide or any other group that can be polymerized into an arylamide gel.

**14.** The kit according to any one of claims 11 to 12, wherein the kit comprises at least two bridge oligonucleotides bearing landing sites for different target nucleic acids.

**15.** The kit according to any one of claims 11 to 14, wherein the anchor oligonucleotide comprises a nucleic acid sequence selected from the group consisting of SEQ ID NO: 1, 2, 3, 4, 5,8, 11, and the bridge oligonucleotides comprises a nucleic acid sequence selected from the group consisting of SEQ ID NO: 6, 7, 9, 10, 12, 13, 14.

Figure 1

**Figure 2**

**EP 4 763 992 A1**

# Figure 3

20

Figure 4

**Figure 5**

**Figure 6**

A

IVT

Bridge

Anchor

CyS    GelRed    Merge

B

Anchor

OligoPool

5S  Oligo

5.8S Oligo

18S Oligo

28S Oligo

1    2    3    4    5    6    7    8

C

total RNA

Bridge

Anchor

6000
4000
3000
2000
1500
1000

# Figure 7

| | Europäisches Patentamt<br>European Patent Office<br>Office européen des brevets | **EUROPEAN SEARCH REPORT** | **Application Number**<br>EP 24 22 2779 |

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KRIEG ELISHA ET AL: "A smart polymer for sequence-selective binding, pulldown, and release of DNA targets", COMMUNICATIONS BIOLOGY, vol. 3, no. 1, 10 July 2020 (2020-07-10), pages 1-9, XP093276097, ISSN: 2399-3642, DOI: 10.1038/s42003-020-1082-2 Retrieved from the Internet: URL:https://www.nature.com/articles/s42003 -020-1082-2> * the whole document * * figures 1d, 2 * * page 2, column 1, paragraph 3 - page 5, column 2, paragraph 1 *<br>----- | 11-15 | INV.<br>C12Q1/6806 |
| X | US 8 034 911 B2 (GENERA BIOSYSTEMS LTD [AU]) 11 October 2011 (2011-10-11) * table 1 * * claims 1-9 * * examples 1-2 * * page 3, lines 41-44 * * page 12, lines 43-62 * * figure 4 *<br>----- | 11-15 | |
| A | REHMAN F ET AL: "Immobilization of acrylamide-modified oligonucleotides by co-polymerization", NUCLEIC ACIDS RESEARCH, OXFORD UNIVERSITY PRESS, GB, vol. 27, no. 2, 15 February 1999 (1999-02-15), pages 649-655, XP002155808, ISSN: 0305-1048, DOI: 10.1093/NAR/27.2.649 * the whole document * * figure 1 *<br>----- | 1-15 | |

TECHNICAL FIELDS SEARCHED (IPC)

C12Q

-/--

The present search report has been drawn up for all claims

| Place of search | Date of completion of the search | Examiner |
|---|---|---|
| The Hague | 10 June 2025 | Bruma, Anja |

EPO FORM 1503 03.82 (P04C01)

page 1 of 2

EUROPEAN SEARCH REPORT

Europäisches
Patentamt

European
Patent Office

Office européen
des brevets

Application Number

EP 24 22 2779

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 411 340 A2 (EXACT SCIENCES CORP [US]) 21 April 2004 (2004-04-21) <br> * paragraphs [0005] - [0012], [0030] - [0038], [0048], [0059] * <br> * claims 1-10 * <br> * examples 1-3 * <br> ----- | 1-15 | |
| A | US 2005/079519 A1 (BOLES TRUETT C [US] ET AL) 14 April 2005 (2005-04-14) <br> * figures 1-3 * <br> * paragraphs [0076] - [0098] * <br> * claims 99-160 * <br> ----- | 1-15 | |
| A | US 2003/175709 A1 (MURPHY GEORGE L [US] ET AL) 18 September 2003 (2003-09-18) <br> * paragraph [0135] * <br> * claims 1-56 * <br> * figure 1 * <br> ----- | 1-15 | |

TECHNICAL FIELDS
SEARCHED      (IPC)

The present search report has been drawn up for all claims

| Place of search | Date of completion of the search | Examiner |
|---|---|---|
| The Hague | 10 June 2025 | Bruma, Anja |

EPO FORM 1503 03.82 (P04C01)

## ANNEX TO THE EUROPEAN SEARCH REPORT
## ON EUROPEAN PATENT APPLICATION NO.

EP 24 22 2779

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2025

| Patent document cited in search report | | Publication date | Patent family member(s) | | Publication date |
|---|---|---|---|---|---|
| US 8034911 | B2 | 11-10-2011 | NZ | 536909 A | 30-06-2006 |
| | | | US | 2006063925 A1 | 23-03-2006 |
| | | | US | 2010261891 A1 | 14-10-2010 |
| | | | WO | 03102228 A1 | 11-12-2003 |
| EP 1411340 | A2 | 21-04-2004 | NONE | | |
| US 2005079519 | A1 | 14-04-2005 | AT | E335845 T1 | 15-09-2006 |
| | | | AU | 730491 B2 | 08-03-2001 |
| | | | CA | 2289925 A1 | 19-11-1998 |
| | | | EP | 0981647 A1 | 01-03-2000 |
| | | | JP | 4276301 B2 | 10-06-2009 |
| | | | JP | 2001525676 A | 11-12-2001 |
| | | | US | 2005079519 A1 | 14-04-2005 |
| | | | US | 2009152116 A1 | 18-06-2009 |
| | | | WO | 9851823 A1 | 19-11-1998 |
| US 2003175709 | A1 | 18-09-2003 | AU | 2002359789 A1 | 09-07-2003 |
| | | | CA | 2468854 A1 | 03-07-2003 |
| | | | EP | 1463835 A2 | 06-10-2004 |
| | | | US | 2003175709 A1 | 18-09-2003 |
| | | | WO | 03054162 A2 | 03-07-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6214187 B1

### Non-patent literature cited in the description

- **M. CHENG et al.** Methods for isolation of messenger RNA from biological samples. *Anal. Methods*, 2021, vol. 13, 289-298

- **STEFANIE KELLNER ; ANTONIA OCHEL ; KATHRIN THÜRING ; FELIX SPENKUCH ; JENNIFER NEUMANN ; SUNNY SHARMA ; KARL-DIETER ENTIAN ; DIRK SCHNEIDER ; MARK HELM**. Absolute and relative quantification of RNA modifications via biosynthetic isotopomers. *Nucleic Acids Research*, 13 October 2014, vol. 42, e142